# EUROPEAN PATENT SPECIFICATION

(11) **EP 3 268 002 B1**
(45) Date of publication and mention of the grant of the patent: **19.08.2020**
(21) Application number: 17720878.2
(22) Date of filing: 31.03.2017
(51) Int. Cl.: A61K 31/497, A61K 31/43, A61P 31/04

(54) **ANTIBACTERIAL COMPOSITIONS AND METHODS**
ANTIBAKTERIELLE ZUSAMMENSETZUNGEN UND VERFAHREN
COMPOSITIONS ANTIBACTÉRIENNES ET MÉTHODES

(30) Priority: 31.03.2016 IN 201621011248
(43) Date of publication of application: 17.01.2018
(73) Proprietor: Wockhardt Limited, M.S. Aurangabad 431006 (IN)
(72) Inventor: PATEL, Mahesh Vithalbhai, Aurangabad 431 003 Maharashtra (IN); BHAGWAT, Sachin, Aurangabad - 431005 Maharashtra (IN); TAKALKAR, Swapna Shripad, Aurangabad - 431005 Maharashtra (IN); CHAVAN, Rajesh, Aurangabad - 431006 Maharashtra (IN); UMARKAR, Kushal, Aurangabad Maharashtra (IN)
(74) Representative: Nederlandsch Octrooibureau
(86) International application number: PCT/IB2017/051873
(87) International publication number: WO 2017/168395

(56) References cited:
- WO-A1-2012/164358
- WO-A2-2007/129176
- CN-B- 102 743 388
- FUBARA J O ET AL: "Influence of pH, temperature and buffers on cefepime degradation kinetics and stability predictions in aqueous solutions", JOURNAL OF PHARMACEUTICAL SCIENCES, vol. 87, no. 12, 1 December 1998 (1998-12-01), pages 1572-1576, XP002628079, AMERICAN PHARMACEUTICAL ASSOCIATION, WASHINGTON, US ISSN: 0022-3549, DOI: 10.1021/JS980170Y [retrieved on 2000-06-08]
- GHAFUR ET AL: "Clinical profile of patients treated with cefepime/tazobactam: A new beta-lactam/beta-lactamase inhibitor combination", J MICROBIOL. INFECT. DIS., vol. 2, no. 3, September 2012 (2012-09), pages 79-86, XP002771409, DOI: 10.5799/ahinjs.02.2012.03.0049

## Description

### RELATED PATENT APPLICATIONS

This application claims priority to and benefit of the Indian Patent Application No. 201621011248 filed on March 31, 2016,

### FIELD OF THE INVENTION

The invention relates to antibacterial compositions and methods for treatment, control or prevention of bacterial infections.

### BACKGROUND OF THE INVENTION

Infections caused by bacteria continue to remain an area of serious concern worldwide. One of the key challenges in the treatment, control or prevention of bacterial infections is the ability of bacteria to develop resistance to one or more antibacterial agents over time. Representative examples of such bacteria that have developed resistance to typical antibacterial agents include: Penicillin-resistant *Streptococcus pneumoniae,* Vancomycin-resistant *Enterococci,* and Methicillin-resistant *Staphylococcus aureus.* The problem of emerging drug-resistance in bacteria is often tackled by switching over to newer antibacterial agents. However, development of new antibacterial agents can be expensive and may not be always a permanent solution as bacteria often develop resistance to the newer antibacterial agents in due course. In general, bacteria are often efficient in developing resistance to antibacterial agent because of their ability to multiply very rapidly and pass on the resistance genes as they replicate. Bacteria develop resistance to existing antibacterial agents through various mechanisms including production of beta lactamases, mutations in the target PBPs, development of efflux pumps, and decreased expression of outer membrane proteins or porins. For example, in response to the continued exposure to a variety of beta-lactam antibacterial agents, bacteria have developed several types of beta lactamases that are capable of hydrolyzing antibacterial agents belonging to penicillins, cephalosporins, monobactams and even carbapenems.

There is an urgent need for development of newer ways to treat bacterial infections, and in particular, infections caused by bacteria that have acquired resistance to one or more of the existing antibacterial agents. A composition comprising at least one antibacterial agent and tazobactam was disclosed in PCT International Patent Application No. PCT/IB2011/053398. For example, a composition comprising cefepime and tazobactam exhibited a synergistic antibacterial effect against a wide variety of bacteria. However, a combination of cefepime and tazobactam when administered intravenously caused inflammation of veins (the effect also known as phlebitis). The inventors have now surprisingly discovered that it is possible to use a composition comprising cefepime and tazobactam parenterally without causing phlebitis by adding a specific amount of arginine or a pharmaceutically acceptable thereof to the composition, and also by using a specific administration regime. The specific amount of each ingredient in the composition, and the administration regime was surprisingly found to result in unexpected synergy, thereby making such compositions and/or therapy effective against several resistant bacteria.

### SUMMARY OF THE INVENTION

The details of one or more embodiments of the invention are set forth in the description below. Other features, objects and advantages of the invention will be apparent from the following description, including claims.

### DETAILED DESCRIPTION OF THE INVENTION

Reference will now be made to the exemplary embodiments, and specific language will be used herein to describe the same. It should nevertheless be understood that no limitation of the scope of the invention is thereby intended. Alterations and further modifications of the inventive features illustrated herein, and additional applications of the principles of the invention as illustrated herein, which would occur to one of ordinary skills in the relevant art and having possession of this disclosure, are to be considered within the scope of the invention. It must be noted that, as used in this specification and the appended claims, the singular forms "a", "an", and "the" include plural referents unless the content clearly dictates otherwise.

The inventors have now surprisingly discovered that it is possible to use a composition comprising cefepime and tazobactam parenterally without causing phlebitis by adding a specific amount of arginine or a pharmaceutically acceptable thereof to the composition, and also by using a specific administration regime. The specific amount of each ingredient in the composition, and the administration regime was surprisingly found to result in unexpected synergy, thereby making such compositions and/or therapy effective against several resistant bacteria.

The term "pharmaceutically acceptable salt" as used herein refers to one or more salts of a given compound which possesses the desired pharmacological activity of the free compound and which are neither biologically nor otherwise undesirable. In general, the "pharmaceutically acceptable salts" refer to and include those salts that are suitable for use in contact with the tissues of human and animals without undue toxicity, irritation, allergic response and the like, and are commensurate with a reasonable benefit/risk ratio. Pharmaceutically acceptable salts are well known in the art. For example, S. M. Berge, et al. (J. Pharmaceutical Sciences, 66: 1-19 (1977)), describes various pharmaceutically acceptable salts in details.

The term "infection" or "bacterial infection" as used herein refers to and includes presence of bacteria, in or on a subject, which, if its growth were inhibited, would result in a benefit to the subject. As such, the term "infection" in addition to referring to the presence of bacteria also refers to normal flora, which is not desirable. The term "infection" includes infections caused by bacteria.

The term "treat", "treating" or "treatment" as used herein refers to administering a medicament, including a pharmaceutical composition, or one or more active ingredients, for prophylactic and/or therapeutic purposes. The term "prophylactic treatment" refers to treating a subject who is not yet infected, but who is susceptible to, or otherwise at a risk of infection (preventing the bacterial infection). The term "therapeutic treatment" refers to administering treatment to a subject already suffering from infection. The terms "treat", "treating" or "treatment" as used herein also refer to administering compositions or one or more of active ingredients discussed herein, with or without additional active or inert ingredients, in order to: (i) reduce or eliminate either a bacterial infection or one or more symptoms of the bacterial infection, or (ii) retard the progression of a bacterial infection or of one or more symptoms of the bacterial infection, or (iii) reduce the severity of a bacterial infection or of one or more symptoms of the bacterial infection, or (iv) suppress the clinical manifestation of a bacterial infection, or (v) suppress the manifestation of adverse symptoms of the bacterial infection.

The term "pharmaceutically effective amount" or "therapeutically effective amount" or "effective amount" as used herein refers to an amount, which has a therapeutic effect or is the amount required to produce a therapeutic effect in a subject. For example, a therapeutically or pharmaceutically effective amount of an active ingredient or a pharmaceutical composition is the amount of the active ingredient or the pharmaceutical composition required to produce a desired therapeutic effect as may be judged by clinical trial results, model animal infection studies, and/or in vitro studies (e.g. in agar or broth media). The pharmaceutically effective amount depends on several factors, including but not limited to, the microorganism (e.g. bacteria) involved, characteristics of the subject (for example height, weight, sex, age and medical history), severity of infection and the particular type of the antibacterial agent or active ingredient used. For prophylactic treatments, a therapeutically or prophylactically effective amount is that amount which would be effective in preventing a microbial (e.g. bacterial) infection. The active ingredients and/or pharmaceutical compositions according to the invention are used in amounts that are effective in providing the desired therapeutic effect or result.

The term "administration" or "administering" includes delivery of a composition, or one or more of active ingredients to a subject, including for example, by any appropriate methods, which serves to deliver the composition or the active ingredients to the site of the infection. The method of administration may vary depending on various factors, such as for example, the components of the pharmaceutical composition or the nature of the active and/or inert ingredients, the site of the potential or actual infection, the microorganism involved, severity of the infection, age and physical condition of the subject and a like. Some non-limiting examples of ways to administer a composition or active ingredients to a subject according to the invention include oral, intravenous, topical, intra-respiratory, intra-peritoneal, intra-muscular, parenteral, sublingual, transdermal, intranasal, aerosol, intra-ocular, intra-tracheal, intra-rectal, vaginal, gene gun, dermal patch, eye drop, ear drop or mouthwash. In case of a pharmaceutical composition comprising more than one ingredient (active or inert), one way to administering such composition is by admixing the ingredients (e.g. in the form of a suitable unit dosage form such as tablet, capsule, solution, powder and a like) and then administering the dosage form. Alternatively, the ingredients may also be administered separately (simultaneously or one after the other) as long as these ingredients reach beneficial therapeutic levels such that the desired therapeutic effect is achieved.

The term "parenteral administration" refers to and includes a route of administration that does not involve gastrointestinal tract directly. Typical, non-limiting examples of parenteral route of administration includes intravenous (into a vein), intra-arterial (into an artery), intraosseous infusion (into the bone marrow), intra-muscular, intracerebral, intrathecal, subcutaneous administration. In general, the parenteral administration is performed by injecting or infusing the composition or the active ingredient(s) directly into a subject without direct involvement of the gastrointestinal tract.

The term "growth" as used herein refers to a growth of one or more microorganisms and includes reproduction or population expansion of the microorganism (e.g. bacteria). The term "growth" also includes maintenance of on-going metabolic processes of a microorganism (e.g. bacteria), including processes that keep the microorganism alive.

The term, "effectiveness" as used herein refers to the ability of a treatment or a composition or one or more active ingredients to produce a desired biological effect in a subject. For example, the term "antibacterial effectiveness" of a composition or an antibacterial agent refers to the ability of the composition or the antibacterial agent to treat or prevent the microbial (e.g. bacterial) infection in a subject.

The term "synergistic" or "synergy" as used herein refers to the interaction of two or more agents so that their combined effect is greater than their individual effects.

The term "antibacterial agent" as used herein refers to any substance, compound or a combination of substances or a combination of compounds capable of: (i) inhibiting, reducing or preventing growth of bacteria; (ii) inhibiting or reducing ability of a bacteria to produce infection in a subject; or (iii) inhibiting or reducing ability of bacteria to multiply or remain infective in the environment. The term "antibacterial agent" also refers to a compound capable of decreasing infectivity or virulence of bacteria.

The term "beta-lactam antibacterial agent" as used herein refers to compounds with antibacterial properties and containing a beta-lactam nucleus in their molecular structure.

The term "beta-lactamase" as used herein refers to any enzyme or protein or any other substance that breaks down a beta-lactam ring. The term "beta-lactamase" includes enzymes that are produced by bacteria and have the ability to hydrolyze the beta-lactam ring in a beta-lactam compound, either partially or completely.

The term "beta-lactamase inhibitor" as used herein refers to a compound capable of inhibiting activity of one or more beta-lactamase enzymes, either partially or completely.

The term "pharmaceutically inert ingredient" or "inert ingredient", "carrier" or "excipient" refers to a compound or material used to facilitate administration of a compound, including for example, to increase the solubility of the compound. Typical, non-limiting examples of solid carriers include, starch, lactose, di-calcium phosphate, sucrose, and kaolin and so on. Typical, non-limiting examples of liquid carriers include sterile water, saline, buffers, non-ionic surfactants, and edible oils such as oil, peanut and sesame oils and so on. In addition, various adjuvants commonly used in the art may be included. These and other such compounds are described in the literature, for example, in the Merck Index (Merck & Company, Rahway, N.J.). Considerations for inclusion of various components in pharmaceutical compositions are described, for example, in Gilman et al. (Eds.) (1990); Goodman and Gilman's: The Pharmacological Basis of Therapeutics, 8th Ed., Pergamon Press.,

The term "subject" as used herein refers to a vertebrate or invertebrate, including a mammal. The term "subject" includes human, animal, a bird, a fish, or an amphibian. Typical, non-limiting examples of a "subject" includes humans, cats, dogs, horses, sheep, bovine cows, pigs, lambs, rats, mice and guinea pigs.

A person of skills in the art would appreciate that the compounds described herein can generally exist or used in various pharmaceutically acceptable forms including in the form of their pharmaceutically acceptable salts, pro-drugs, metabolites, esters, ethers, hydrates, polymorphs, solvates, complexes, enantiomers, adducts or such other pharmaceutically acceptable derivatives. A reference to the compound, therefore, is intended to include it's pharmaceutically acceptable salts, pro-drugs, metabolites, esters, ethers, hydrates, polymorphs, solvates, complexes, enantiomers, adducts or such other pharmaceutically acceptable derivative. For example, the terms "cefepime", "tazobactam", or "arginine" includes their pharmaceutically acceptable salts, pro-drugs, metabolites, esters, ethers, hydrates, polymorphs, solvates, complexes, enantiomers, adducts or such other pharmaceutically acceptable derivatives. Each of cefepime or a pharmaceutically acceptable salt thereof, tazobactam or a pharmaceutically acceptable salt thereof, and arginine or a pharmaceutically acceptable salt thereof, is individually referred to as an "active ingredient" and collectively referred to as "active ingredients". The terms "pharmaceutical compositions" or "composition" as used herein refer to and include the compositions according to the invention.

In some embodiments, there is provided a method for treating bacterial infection in a subject by parenteral administration of active ingredients, said active ingredients comprising: (a) 2 gram of cefepime or a pharmaceutically acceptable salt thereof, (b) 0.70 to 0.80 gram arginine or a pharmaceutically acceptable salt thereof, per gram of cefepime or a pharmaceutically acceptable salt thereof, and (c) 2 gram of tazobactam or a pharmaceutically acceptable salt thereof; said method comprising that the active ingredients are administered parenterally over a period of about 15 minutes to about 24 hours.

In some other embodiments, there is provided a method for treating bacterial infection in a subject by parenteral administration of active ingredients in any of the following amounts over a period of about 15 minutes to about 250 minutes:
(i) (a) 2 gram of cefepime or a pharmaceutically acceptable salt thereof, (b) 0.70 to 0.80 gram arginine or a pharmaceutically acceptable salt thereof, per gram of cefepime or a pharmaceutically acceptable salt thereof, and (c) 2 gram of tazobactam or a pharmaceutically acceptable salt thereof.

In some other embodiments, there is provided a method for treating bacterial infection in a subject by parenteral administration of active ingredients in any of the following amounts over a period of about 30 minutes to about 120 minutes:
(i) (a) 2 gram of cefepime or a pharmaceutically acceptable salt thereof, (b) 0.70 to 0.80 gram arginine or a pharmaceutically acceptable salt thereof, per gram of cefepime or a pharmaceutically acceptable salt thereof, and (c) 2 gram of tazobactam or a pharmaceutically acceptable salt thereof.

In some other embodiments, there is provided a method for treating bacterial infection in a subject by parenteral administration of active ingredients in any of the following amounts over a period of about 30 minutes to about 90 minutes:
(i) (a) 2 gram of cefepime or a pharmaceutically acceptable salt thereof, (b) about 0.70 to 0.80 gram arginine or a pharmaceutically acceptable salt thereof, per gram of cefepime or a pharmaceutically acceptable salt thereof, and (c) 2 gram of tazobactam or a pharmaceutically acceptable salt thereof.

In some embodiments, the active ingredients are administered simultaneously or one after the other.

In some other embodiments, the active ingredients are administered in the form of a solution, the solution being obtained by adding the active ingredients to a compatible liquid diluent.

In some embodiments, each of the active ingredients is administered independently in the form of a solution, the solution being obtained by dissolving the active ingredient in a compatible liquid diluent.

A wide variety of liquid diluents can be used. Typical, non-limiting example of liquid diluent includes water for injection, 0.9% sodium chloride solution, 5% dextrose solution, normal saline solution and a like.

In some embodiments the active ingredients according to the invention are administered in the form of a solution as described herein, said solution having a pH within a range between about 6.5 to about 8.

The active ingredients according to the invention can be administered at varied time intervals depending upon the specific requirement or the desired therapeutic effect. In some embodiments, the active ingredients according to the invention are administered one, two, three or four times a day. In some other embodiments, the active ingredients according to the invention are administered every 4 hours, 6 hours, 8 hours, 12 hours or 24 hours.

The active ingredients according to the invention may also be administered in combination with one or more pharmaceutically acceptable carriers or excipients or inert ingredients. Typical, non-limiting examples of such carriers or excipients or inert ingredients include mannitol, lactose, starch, magnesium stearate, sodium saccharine, talcum, cellulose, sodium crosscarmellose, glucose, gelatin, sucrose, magnesium carbonate, wetting agents, emulsifying agents, solubilizing agents, buffering agents, lubricants, stabilizing agents, binding agents and a like.

In some embodiments, the active ingredients according to the invention are administered in combination with one or more buffering agent. Typical non-limiting examples of buffering agents include aluminum hydroxide, aluminum hydroxide/magnesium carbonate co-precipitate, aluminum hydroxide/sodium bicarbonate co-precipitate, aluminium glycinate, aluminium magnesium hydroxide, aluminium phosphate, calcium acetate, calcium carbonate, calcium formate, calcium bicarbonate, calcium borate, calcium citrate, calcium gluconate, calcium glycerophosphate, calcium hydroxide, calcium chloride, calcium lactate, calcium phthalate, calcium phosphate, calcium succinate, calcium tartrate, calcium propionate, dibasic sodium phosphate, dipotassium hydrogen phosphate, dipotassium phosphate, disodium hydrogen phosphate, disodium succinate, dry aluminum hydroxide gel, magnesium acetate, magnesium aluminate, magnesium borate, magnesium bicarbonate, magnesium hydroxide, magnesium carbonate, magnesium citrate, magnesium gluconate, magnesium lactate, magnesium metasilicate aluminate, magnesium oxide, magnesium phthalate, magnesium phosphate, magnesium silicate, magnesium succinate, magnesium tartrate, potassium acetate, potassium carbonate, potassium bicarbonate, potassium borate, potassium citrate, potassium metaphosphate, potassium phthalate, potassium phosphate, potassium polyphosphate, potassium pyrophosphate, potassium succinate, potassium tartrate, sodium acetate, sodium bicarbonate, sodium borate, sodium carbonate, sodium citrate, sodium gluconate, sodium hydrogen phosphate, sodium hydroxide, sodium lactate, sodium phthalate, sodium phosphate, sodium polyphosphate, sodium pyrophosphate, sodium sesquicarbonate, sodium succinate, sodium tartrate, sodium tripolyphosphate, synthetic hydrotalcite, tetrapotassium pyrophosphate, tetrasodium pyrophosphate, tripotassium phosphate, trisodium phosphate, trometamol, trihydroxymethylaminomethane, an amino acid such as alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, valine or the optically active isomers thereof, or the racemic mixtures thereof, an acid salt of an amino acid, an alkali slat of an amino acid or mixtures thereof.

In another general aspect, there are provided methods for treatment, control or prevention of bacterial infection using the active ingredients according to the invention. In some embodiments, there is provided a method for treatment, control or prevention of bacterial infection in a subject, said method comprising administering to said subject an effective amount of active ingredients according to the invention.

In some embodiments, the active ingredients according to the invention are used in treatment, control or prevention of bacterial infection.

In other embodiments, the active ingredients according to the invention are administered by any appropriate method, which serves to deliver the composition or its constituents to the desired site. In case of methods using administration of active ingredients, the active ingredients may also be administered by any appropriate method. The method of administration can vary depending on various factors, such as for example, the nature of the active ingredients or the composition, the site of the potential or actual infection, the microorganism involved, severity of infection, age and physical condition of the subject and so on. In some embodiments, the active ingredients according to the invention are administered parenterally. Some non-limiting examples of administration methods according to the invention include intravenous, intraperitoneal, intramuscular, parenteral, intratracheal, intrarectal and a like.

In another general aspect, the active ingredients according to the invention are used in prophylactic treatment of a subject, comprising administering to a subject at risk of infection caused by bacteria, a prophylactically effective amount the active ingredients according to the invention.

In general, the active ingredients according to the invention are effective against infections caused by a wide variety of bacteria, including those exhibiting resistance to one or more of known antibacterial agents or compositions. Some non-limiting examples of infections that can be treated, controlled or prevented using the compositions and methods according the invention include infections caused by bacteria belonging to genus *Escherichia, Staphylococcus, Streptococcus, Haemophilus, Klebsiella, Moraxella, Enterobacter, Proteus, Serratia, Pseudomonas, Acinetobacter, Citrobacter, Stenotrophomonas, Bacteroides, Prevotella, Fusobacterium, Clostridium.*

In general, the active ingredients according to the invention are useful in treatment, control or prevention of several infections, including, for example, skin and soft tissue infections, febrile neutropenia, urinary tract infections, intraabdominal infections, respiratory tract infections, pneumonia (nosocomial), bacteremia, meningitis, diabetic foot infections, bone and joint infections, surgical site infections, Shigella dysentery and the like.

### EXAMPLES

The following examples illustrate the embodiments of the invention that are presently best known. However, it is to be understood that the following are only exemplary or illustrative of the application of the principles of the present invention. Thus, while the present invention has been described above with particularity, the following examples provide further detail in connection with what are presently deemed to be the most practical and preferred embodiments of the invention

The efficacy of methods according to the invention was investigated using neutropenic murine lung infection model at doses which are corresponding to clinical doses of 2 gram of cefepime and 2 gram of tazobactam q8h, and 30 to 90 min infusion. The protocol employed is a widely employed approach to predict clinical efficacy of a given dosing regimen through mouse models of infection. In a typical study, male and female Swiss albino mice weighing 25-27 gram were rendered neutropenic with 150 and 100 mg/kg intraperitoneal injections of cyclophosphamide (Endoxan-Asta) given 1 and 4 day prior to infection. Two hours prior to the initiation of antimicrobial therapy, mice were instilled via intranasal route with 80 µl of bacterial suspension, containing approximately 10⁶ log10 CFU/ml of infecting pathogen (K. pneumoniae H524). Beginning 2 hours after infection, groups of 6 mice were administered (subcutaneous route) humanized doses of the cefepime and tazobactam. All doses were administered as 0.250 ml subcutaneous injections at a frequency of q1h (every one hour) and q2h (2 hourly) dosing intervals over 24 hours. Lungs from all the animals including untreated animals were harvested 3 hours post last dose of regimen and individually homogenized in 3 ml normal saline. Bacterial count in homogenized lungs were determined and expressed in colony forming unit (CFU) per lung. Bacterial load in lungs of untreated animals enumerated at the time of initiation of therapy (2 hours post infections) served as reference count to quantify the magnitude of antibacterial effect realized through applied dosing regimens.

| Cefepime + tazobactam dose (mg/kg) | Dosing regimen | Total daily dose (mg/kg) | Corresponding clinical dose |
|---|---|---|---|
| 50 + 50 | q1h | 1200 | 2 gram of cefepime + 2 gram of tazobactam (90 min infusion) |
| 100 + 100 | q2h | 1200 | 2 gram of cefepime + 2 gram of tazobactam (30 min infusion) |

To mimic 90 min infusion in human, cefepime + tazobactam combination was administered every 1 hour over 24 hours. Similarly, to mimic 30 min infusion in human, the combination was administered every 2 hours over 24 hours. The results obtained showed that, at a dose of 50 mg/kg q1h mimicking 90 min infusion in humans resulted into 4.5 log kill of bacteria per lung over initial bacterial lung load. While 100 mg/kg q2h mimicking 30 min infusion resulted into just 1.9 log kill. The improvement in bactericidal effect by 90 min infusion is better than that obtained with a 30 min infusion.

## Claims

1. Active ingredients for use in a method for treating bacterial infection in a subject, said active ingredients comprising: (a) 2 gram of cefepime or a pharmaceutically acceptable salt thereof, (b) 0.70 to 0.80 gram of arginine or a pharmaceutically acceptable salt thereof, per gram of cefepime or the pharmaceutically acceptable salt thereof, and (c) 2 gram of tazobactam or a pharmaceutically acceptable salt thereof; said method comprising that the active ingredients are administered parenterally over a period of 15 minutes to 250 minutes.

2. The active ingredients for use according to Claim 1, wherein the active ingredients are administered parenterally over a period of 30 minutes to 120 minutes.

3. The active ingredients for use according to Claim 1, wherein the active ingredients are administered parenterally over a period of 30 minutes to 90 minutes.

4. The active ingredients for use according to Claim 1, wherein the active ingredients are administered parenterally over a period of 90 minutes to 120 minutes.

5. The active ingredients for use according to Claim 1, wherein the active ingredients are administered parenterally over a period of 90 minutes.

6. The active ingredients for use according to any one of Claims 1 to 5, wherein the ingredients are administered simultaneously or one after the other.

7. The active ingredients for use according to any one of Claims 1 to 6, wherein the active ingredients are administered one, two, three or four times a day.

8. The active ingredients for use according to any one of Claims 1 to 6, wherein the active ingredients are administered every 6 hours, 8 hours, 12 hours or 24 hours.

## Patentansprüche

1. Wirkstoffe zur Verwendung in einem Verfahren zur Behandlung einer bakteriellen Infektion bei einem Patienten, wobei die Wirkstoffe umfassen: (a) 2 Gramm Cefepim oder ein pharmazeutisch annehmbares Salz davon, (b) 0,70 bis 0,80 Gramm Arginin oder ein pharmazeutisch annehmbares Salz davon pro Gramm Cefepim oder seines pharmazeutisch annehmbaren Salzes und (c) 2 Gramm Tazobactam oder ein pharmazeutisch annehmbares Salz davon; wobei das Verfahren umfasst, dass die Wirkstoffe über einen Zeitraum von 15 Minuten bis 250 Minuten parenteral verabreicht werden.

2. Wirkstoffe zur Verwendung gemäß Anspruch 1, wobei die Wirkstoffe über einen Zeitraum von 30 Minuten bis 120 Minuten parenteral verabreicht werden.

3. Wirkstoffe zur Verwendung gemäß Anspruch 1, wobei die Wirkstoffe über einen Zeitraum von 30 Minuten bis 90 Minuten parenteral verabreicht werden.

4. Wirkstoffe zur Verwendung gemäß Anspruch 1, wobei die Wirkstoffe über einen Zeitraum von 90 Minuten bis 120 Minuten parenteral verabreicht werden.

5. Wirkstoffe zur Verwendung gemäß Anspruch 1, wobei die Wirkstoffe über einen Zeitraum von 90 Minuten parenteral verabreicht werden.

6. Wirkstoffe zur Verwendung gemäß einem der Ansprüche 1 bis 5, wobei die Wirkstoffe gleichzeitig oder nacheinander verabreicht werden.

7. Wirkstoffe zur Verwendung gemäß einem der Ansprüche 1 bis 6, wobei die Wirkstoffe ein-, zwei-, drei- oder viermal am Tag verabreicht werden.

8. Wirkstoffe zur Verwendung gemäß einem der Ansprüche 1 bis 6, wobei die Wirkstoffe alle 6 Stunden, 8 Stunden, 12 Stunden oder 24 Stunden verabreicht werden.

## Revendications

1. Ingrédients actifs destinés à être utilisés dans une méthode de traitement d'une infection bactérienne chez un sujet, lesdits ingrédients actifs comprenant : (a) 2 grammes de céfépime ou d'un sel pharmaceutiquement acceptable de celle-ci, (b) 0,70 à 0,80 gramme d'arginine ou d'un sel pharmaceutiquement acceptable de celle-ci, par gramme de céfépime ou du sel pharmaceutiquement acceptable de celle-ci, et (c) 2 grammes de tazobactam ou d'un sel pharmaceutiquement acceptable de celui-ci ; ladite méthode comprenant que les ingrédients actifs sont administrés par voie parentérale sur une période de 15 minutes à 250 minutes.

2. Ingrédients actifs à utiliser selon la revendication 1, dans lesquels les ingrédients actifs sont administrés par voie parentérale sur une période de 30 minutes à 120 minutes.

3. Ingrédients actifs à utiliser selon la revendication 1, dans lesquels les ingrédients actifs sont administrés par voie parentérale sur une période de 30 minutes à 90 minutes.

4. Ingrédients actifs à utiliser selon la revendication 1, dans lesquels les ingrédients actifs sont administrés par voie parentérale sur une période de 90 minutes à 120 minutes.

5. Ingrédients actifs à utiliser selon la revendication 1, dans lesquels les ingrédients actifs sont administrés par voie parentérale sur une période de 90 minutes.

6. Ingrédients actifs à utiliser selon l'une quelconque des revendications 1 à 5, dans lesquels les ingrédients sont administrés simultanément ou les uns après les autres.

7. Ingrédients actifs à utiliser selon l'une quelconque des revendications 1 à 6, dans lesquels les ingrédients actifs sont administrés une, deux, trois ou quatre fois par jour.

8. Ingrédients actifs à utiliser selon l'une quelconque des revendications 1 à 6, dans lesquels les ingrédients actifs sont administrés toutes les 6 heures, 8 heures, 12 heures ou 24 heures.
